# EUROPEAN PATENT SPECIFICATION

(11) **EP 0 722 922 B1**
(45) Date of publication and mention of the grant of the patent: **18.08.1999**
(21) Application number: 95300318.3
(22) Date of filing: 19.01.1995
(51) Int. Cl.: C07C 2/30, C07C 2/36

(54) **Manufacture of linear alpha-olefins**
Verfahren zur Herstellung von linearen alpha-Olefinen
Procédé pour la préparation d'alpha-oléfines linéaires

(43) Date of publication of application: 24.07.1996
(73) Proprietor: INDIAN PETROCHEMICALS CORPORATION LIMITED, District Vadodara 391346 Gujarat (IN)
(72) Inventor: Tembe, Gopal Laxman, District Vadodara 391 346 Gujarat (IN); Bandyopadhyay, Ashis Ranjan, District Vadodara 391 346 Gujarat (IN); Pillai, Subramania Muthukumaru Dr., District Vadodara 391 346 Gujarat (IN); Satish, Sheo, District Vadodara 391 346 Gujarat (IN); Ravindranathan, Marayil Dr., District Vadodara 391 346 Gujarat (IN)
(74) Representative: Marshall, Monica Anne

(56) References cited:
- EP-A- 0 221 206
- EP-A- 0 516 852
- GB-A- 1 231 299
- US-A- 4 855 526
- CHEMICAL ABSTRACTS, vol. 84, no. 3, 19 January 1976 Columbus, Ohio, US; abstract no. 16723h, page 410; & JP-A-50 022 004 (MITSUI PETROCHEMICAL INDUSTRIES) 28 July 1975

## Description

The present invention relates to a process for the manufacture of linear alpha-olefins. More particularly, the invention comprises the manufacture of linear alpha-olefins by oligomerizing ethylene. More particularly, the invention relates to the manufacture of alpha-olefins by oligomerizing ethylene in presence of a catalyst system which is a mixture of
1] titanium aryl oxide/alkoxide;
2] alkyl aluminum halide; and
3] a phosphorus, an oxygen or a sulphur compound.

The process of the invention is useful in producing predominantly highly linear, low molecular weight alpha-olefins.

Alpha olefins in the range of 4-40 carbon numbers are known to be prepared by wax cracking, paraffin dehydrogenation or dehydration of alcohols. However, these processes suffer from the drawback of low conversions, product purity of specific alpha olefins and high energy costs.

Other widely used processes are based on ethylene chain growth and displacement reactions [Ziegler]. These processes utilize trialkyl aluminum, particularly triethylaluminum at high temperatures [170-200°C] and pressures [140 atm]. A particular drawback of such processes is the difficulty in the separation of lighter and higher olefins and the formation of internal and vinylidene olefins.

Several patents known hereto before disclose the catalytic low temperature oligomerization of ethylene based on titanium halide [e.g. TiCl₄], alkyl aluminum halide [e.g. EtAlCl₂][Ziegler-Natta] modified by phosphines [e.g. nBu₃P]. The selectivity of alpha olefins in these cases is affected by copolymerization reactions yielding undesirable branched olefins.

British Patent No. 787,438, issued on December 11, 1957 describes the preparation of lower alpha olefins with less than 6 carbon numbers utilizing a catalyst prepared by combining titanium tetra alkoxide and triethyl aluminum. This process involves moderate ethylene conversions.

Low temperature oligomerization by halogenated titanium alkoxides is disclosed in German Patent No. 1,924,427, issued on April 23, 1970 yielding alpha olefins upto C₅₀. Another class of catalysts which have been utilized are organoaluminum-free compounds [non-Ziegler] [e.g. Ni(COD)₂ or nickel ylides] to convert ethylene to higher oligomers of even carbon numbers [e.g. C₄-C₂₀+] which are linear in character. The non-Ziegler route using bis [cyclooctadiene] nickel [o] or its complexes with fluorinated compounds [e.g. hexafluoroacetyl acetone] is described in the U.S. Patent 3,644,564 issued on February 22, 1972 and commercialized in the Shell Higher Olefins Process [SHOP].

GB 1231299 discloses polymerization of polypropylene at a temperature from -40 to 20°C. In US 4855526 copolymers of ethylene and higher olefins are produced using Ziegler-Natta catalysts. In EP-A-0516852 butene-1 is obtained by dimerizing ethylene. Chemical Abstracts vol 84 no.3, abstract no. 16723h at page 410 discloses the formation of C₈-C₂₀ olefins by providing ethylene with α-olefins of greater than four carbon atoms over certain catalysts.

The primary objective of the present invention is to provide a novel process for producing linear alpha-olefins using a novel catalyst system.

The process results in the production of linear alpha-olefins having 4-36 carbon atoms and particularly, highly selective for the more useful range of C₄-C₂₀.

Another aspect of the invention involves high conversions of the ethylene to low molecular weight product olefins under controlled reaction conditions.

In accordance with the present invention, there is provided a process for the manufacture of linear alpha-olefins which comprises oligomerizing ethylene in an inert organic solvent in the presence of a catalyst mixture comprising titanium aryl/alkyl oxide, organoaluminum halide and optionally an additive selected from a group containing a phosphorus, oxygen or sulphur compound at a temperature of from 25°C to 150°C and at a pressure which is sufficient to maintain the solubility of the ethylene feed and to prevent formation of branched products to produce the desired linear alpha-olefin.

The catalyst employed in the oligomerization process comprises a titanium aryl oxide/alkoxide, organoaluminum halide and another component consisting of a phosphine, phosphite, cyclic ether or a sulphur compound.

With respect to the catalyst titanium alkyl oxide/aryl oxide is represented as Ti[OR]₄ and all the titanium [IV] cresylates [i.e. ortho, meta or para] are preferred. However, a non-limiting list of titanium aryl and alkyl oxides include titanium [IV] 2,6 dimethyl phenoxide and commercial tetra-2-ethyl hexyl titanate, triethanolamine titanate, diethanolamine titanate and the like.

The phosphorus additives in the catalyst of the present invention is preferably selected from the group PPh₃, Ph₂PCH₂CH₂PPh₂, [PhO]₂P[O][OH] and P[nBu]₃.

The oxygenated compound of the preferred catalyst composition is selected from the group consisting of tetrahydrofuran and 1,4 dioxane.

The sulphur compound employed as an additive in the tricomponent catalyst of the present invention is preferably 2-mercaptobenzothiazole.

The titanium to additive molar ratio is conveniently maintained between 0.05 to 0.1.

The catalyst employed in the process of the present invention preferably has the formula

Ti[OR]ₙ[OR']₄₋ₙ-EtₓAl₂Cl₆₋ₓ - L

wherein x = 2, 3 or 4 and L is selected from the group of the additives selected from phosphorus, oxygen or sulphur compound,
when n = 4, R is 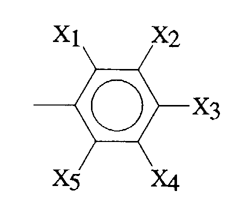 where
X₁ = CH₃; X₂=X₃=X₄=X₅ = H
X₂ = CH₃; X₁=X₃=X₄=X₅ = H
X₃ = CH₃; X₁=X₂=X₄=X₅ = H
X₁ = X₅=CH₃; X₂=X₃=X₄ = H
or
R is 2-ethylhexyl or when n = 2, R is triethanolamino or diethanolamino, R' is isopropyl.

The oligomerization is carried out at a temperature of from 25 to 150°C. A more preferred range is from 50°C-100°C. At reaction temperatures below ambient, internal, branched or polymeric products are preferentially formed. On the other hand, elevation in temperature enhances the formation of linear oligomeric products.

However, no significant change in alpha selectivity is obtained by increasing the temperature beyond the highest specified temperature. Accordingly, temperature is an important reaction parameter determining the distribution and production of alpha-olefins.

During oligomerization ethylene pressures between 140800 and 246400 kg/m² (200 and 350 psi) are preferred. Pressures between 176000 and 211200 kg/m² (250 and 300 psi) are more preferable.

Both aromatic or halogenated aromatic or aliphatic solvents can be employed as the reaction medium. Suitable list of solvents include n-octane, toluene, chlorobenzene, dichloromethane and the like. Toluene is especially preferred as a solvent.

Preferably during ethylene oligomerization titanium aryl oxide or an alkyl oxide and an organoaluminum halide of the catalyst are modified by phosphine, phosphite, a cyclic ether or a sulphur compound.

More preferably, the catalyst solution is prepared by reacting titanium [IV] cresylate, ethylaluminum sesquichloride and with or without P(nBu)₃ thereby enabling the control of ethylene oligomers to predominantly highly linear, low molecular weight product.

The linearity or the alpha-purity is the percentage weight fraction of the alpha olefins in the total olefinic product including those cases where polymer formation was observed.$\text{linearity =} \frac{\text{alpha olefins}}{\text{alpha + internal/branched + polymer}} \text{x 100}$

In order to obtain the desired selectivity all the reaction parameters are taken into consideration such as the composition of the catalyst, molar ratio of catalyst components, ethylene pressure, temperature, nature of solvent and the nature of additives.

The active catalyst solution is preferably prepared in situ just prior to carrying out the oligomerization reaction. When preparing the three component catalyst solution the phosphorus, oxygen or thio compounds are added to the appropriate mixture of the Ti[OR]₄ and organo aluminum halide. These highly active oligomerization catalysts are transferred in the absence of oxygen and moisture to the reactor before charging the feed under pressure.

The preferred mole ratio for catalyst components [e.g. Et₃Al₂Cl₃/Ti[OR]₄ can be from 12 to 18. The alpha olefin selectivity at these ratios is 93 to 99.9% in the C₄ to C₃₆ range. The aluminum to titanium mole ratio can, however, be varied from about 6 to 18.

The process of oligomerization of this invention is suitably carried out in a stainless steel autoclave having provision for heating, cooling and stirring. The preferred reaction time range from about 10 minutes to 1 hour. The batch reactions of this process are generally quenched by injecting 3-5 ml of n-butyl alcohol. The tabulated yields of ethylene oligomers do not correspond to the actual catalyst efficiency and are based on the total weight percent of ethylene converted to products.

The process of the invention will now be described with reference to the following examples.

### EXAMPLE 1

A 300 ml stainless steel reactor [activated with high purity nitrogen for at least 2 hours at 140°C] was charged with 0.804 grams [1.68 m mol], Ti(m-OC₆H₄CH₃)₄ 80 ml toluene and 3.84 grams [0.03 mol] EtAlCl₂. The vessel was pressurized with ethylene at 25°C to 140800 kg/m² (200 psi). The temperature was then increased to 50°C and maintained there for 1 hour by which time the ethylene uptake was complete indicated by pressure drop to approximately 0 psi. Gas sample was collected and the reaction mixture containing the active catalyst was quenched with 3.0 ml n-BuOH. This liquid sample and the gas sample were analysed by gas chromatography showing 90.2 wt % ethylene conversion and selectivity of linear olefins 1-C₄[3.5], 1-C₆[7.8], 1-C₈[10.9], 1-C₁₀ [3.7], 1-C₁₂ [10.1], 1-C₁₄ [3.8], 1-C₁₆ [3.7], 1-C₁₈ [5.2], 1-C₂₀ [4.9], 1-C₂₂ [4.8], 1-C₂₄ [3.2], 1-C₂₆ [2.1], 1-C₂₈ [2.1], 1-C₃₀ [1.4], 1-C₃₂ [1.0], 1-C₃₄ [0.8] internal olefins [13] and polymer [17.4].

### EXAMPLE 2

The method of example 1 was followed: The 300 ml reactor was charged with 0.71 grams, [1.5 m mol], Ti[p-OC₆H₄CH₃]₄, 80 ml toluene and 6.69 grams [0.028 mol] Et₃Al₂Cl₃. The reaction temperature was maintained at 100°C and the ethylene charged to have a pressure of 140800 kg/m² (200 psi) in the vessel. The weight of alpha olefins was 10.9 grams after 1 hour at 99.4 wt % conversion and linear olefin selectivity 1-C₄ [13.9], 1-C₆ [15.9], 1-C₈ [17.4], 1-C₁₀ [7.2], 1-C₁₂ [6.5],1-C₁₄ [5.5], 1-C₁₆ [5.5], 1-C₁₈ [5.0], 1-C₂₀ [4.2], 1-C₂₂ [3.6], 1-C₂₄ [2.9], 1-C₂₆ [2.5], 1-C₂₈ [2.2], 1-C₃₀ [3.3], 1-C₃₂ [1.8], 1-C₃₄ [1.6].

### EXAMPLE 3

The procedure of example 1 was followed: In a 300 ml stainless steel reactor there were placed 0.74 grams [1.40 m mol] Ti[OC₆H₃-2,6-Me₂]₄ 80 ml toluene and 6.2 grams [0.025 mol] Et₃Al₂Cl₃. Ethylene was charged into the vessel to 140800 kg/m² (200 psi) pressure at a reaction temperature of 150°C. The reaction was terminated after 15 minutes and the products analyzed as in example 1. The yield of pure alpha olefins was 8.5 grams at 99.2 wt % conversion and an alpha olefin distribution showing 1-C₄[4.7], 1-C₆ [19.1], 1-C₈ [16.5], 1-C₁₀ [11.3],1-C₁₂ [10.9], 1-C₁₄ [8.8], 1-C₁₆ [7.4], 1-C₁₈ [5.3], 1-C₂₀ [4.3], 1-C₂₂,⁺ [11.4].

### EXAMPLE 4

The procedure of example 1 was followed: In a 300 ml stainless steel reactor there were added a catalyst solution containing 0.685 grams, [1.44 m mol] Ti(p-OC₆H₄CH₃)₄, 80 ml toluene, 0.26 mol Et₃Al₂Cl₃ and 0.14 mol THF. Ethylene was pressurized to 246400 kg/m² (350 psi). After 1 hour reaction at 150°C the total yield of linear alpha olefins was 13.9 grams at 99.4 wt % ethylene conversion. The analysis showed the following alpha olefin selectivity 1-C₄ [5.5], 1-C₆ [8.5], 1-C₈ [17.6], 1-C₁₀ [11.6], 1-C₁₂ [11.4], 1-C₁₄ [9.6], 1-C₁₆ [7.7],1-C₁₈ [6.0], 1-C₂₀ [4.3], C₂₂+ [12.9] and polymer [4.7].

### EXAMPLE 5

For the purpose of comparison the example 1 was followed. Chlorobenzene was employed as the solvent for preparing the catalyst consisting of 0.78 grams, [1.64 m mol] Ti[p-OC₆H₄CH₃]₄ and 7.3 grams [0.029 mol] El₃Al₂Cl₃. Ethylene was then charged to the reactor maintained at 50°C to a pressure of 140800 kg/m² (200 psi). After 1 hour the wt % ethylene conversion was 97.5 and the alpha olefin yield 7.8 grams with the following selectivity 1-C₄ [18.8], 1-C₆ [19.0], 1-C₈ [24.4], 1-C₁₀ [10.5], 1-C₁₂ [8.1], 1-C₁₄ [5.1], 1-C₁₆ [3.7], 1-C₁₈ [2.5], 1-C₂₀ [2.0], 1-C₂₂ ^{+'} [5.5].

The catalyst system employed in the process of the present invention and illustrated in examples 1 to 5 are influenced by the nature of R in Ti[OR]₄, the reaction temperature, cocatalyst/catalyst ratio and the nature of additive. Tables I-III summarize and compare the results of ethylene oligomerization under different conditions.

**TABLE I**

| Ethylene oligomerization with titanium [IV] cresylates | | | | |
|---|---|---|---|---|
| Run No. | Catalyst | Conv. % | α-olefin % ^{C}4^{-C}36 | Polymer % |
| 1. | Ti(o-OC₆H₄CH₃)₄ | 97.8 | 99.8 | 0.01 |
| 2. | Ti(m-OC₆H₄CH₃)₄ | 98.9 | 98.0 | 2.0 |
| 3. | Ti(p-OC₆H₄CH₃)₄ | 98.2 | 99.4 | 0.6 |
| ^{P}C₂H₄ | 140800 kg/m² (200 psi) | | | |
| TEMPERATURE | 100°C | | | |
| COCATALYST | Et₃Al₂Cl₃ | | | |
| SOLVENT | Toluene | | | |
| Cocatalyst/Catalyst molar ratio 18; Reaction time 60 minutes | | | | |

**TABLE II**

| Oligomerization of ethylene with titanium [IV] aryl/alkyloxides | | | | | |
|---|---|---|---|---|---|
| Run No. | Catalyst | Temp. °C | Conv. % | α-olefins % C₄-C₃₆ | Polymer % |
| 1. | Ti(m-OC₆H₄CH₃)₄ | 50 | 98.1 | 98.4 | 1.6 |
| 2. | Ti(OCH₂CH[Et) | 50 | 92.4 | 97.7 | 12.3 |
| | (CH₂)₃Me)₄ | | | | |
| 3. | Triethanolamine | 50 | 98.7 | 99.1 | 0.9 |
| | titanate | | | | |
| 4. | Diethanolamine | 50 | 97.5 | 64.1 | 35.9 |
| | titanate | | | | |
| 5. | Ti(OC₆H₃-2,6-Me₂⁾₄ | 50 | 98.9 | 92.7 | 7.5 |
| ^{P}C₂H₄ | 140800 kg/m² (200 psi) | | | | |
| COCATALYST | Et₃Al₂Cl₃ | | | | |
| SOLVENT | Toluene | | | | |
| Cocatalyst/Catalyst molar ratio 18; Reaction time 60 minutes | | | | | |

**TABLE III**

| Influence of additives on ethylene oligomerization | | | | |
|---|---|---|---|---|
| Run No. | Additive | Conv. % | α-olefin % | Polymer |
| 1 | P(n-Bu)₃ | 99.6 | 99.9 | Nil |
| 2 | PPh₃ | 93.3 | 97.9 | Nil |
| 3 | THF | 99.3 | 95.3 | 4.6 |
| 4 | 1,4 dioxane | 99.9 | 65.3 | 34.7 |
| 5 | 2-mercaptobenzothiazole | 93.1 | 79.9 | 20.1 |
| ^{P}C₂H₄ | 246400 kg/m² (350 psi) | | | |
| TEMPERATURE | 150°c | | | |
| CATALYST | Ti(p-OC₆H₄Me)₄-Et₃Al₂Cl₃; | | | |
| SOLVENT | Toluene | | | |
| Cocatalyst/Catalyst molar ratio 18; Reaction time 60 minutes; | | | | |
| Ti : Additive 0.1 (molar ratio) | | | | |

## Claims

1. A process for the manufacture of a linear alpha-olefin which comprises oligomerizing ethylene in an inert organic solvent in the presence of a catalyst mixture comprising titanium aryl/alkyl oxide, organoaluminum halide and optionally an additive selected from a group containing a phosphorus, oxygen or sulphur compound at a temperature of from 25°C to 150°C and at a pressure which is sufficient to maintain the solubility of the ethylene feed and to prevent formation of branched products to produce the desired linear alpha-olefin.

2. A process as claimed in claim 1 wherein said catalyst has the formula
Ti [OR]ₙ[OR']₄₋ₙ-EtₓAl₂Cl₆₋ₓ - L
wherein
x is 2, 3 or 4,
Et is ethyl,
L is derived from any phosphorus, oxygen or sulphur compound used as additive, and
n = 4, and
R is 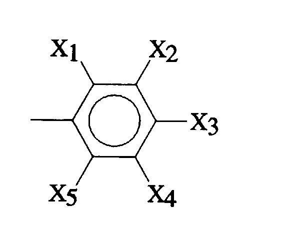
(wherein
X₁ = CH₃; X₂=X₃=X₄=X₅ = H
X₂ = CH₃; X₁=X₃=X₄=X₅ = H
X₃ = CH₃; X₁=X₂=X₄=X₅ = H
X₁ = X₅=CH₃; X₂=X₃=X₄ = H)
or
R is 2-ethylhexyl, or
n = 2, and R is triethanolamino or diethanolamino, and R' is isopropyl.

3. A process as claimed in either of the preceding claims wherein the oligomerization temperature is from 50°C-150°C.

4. A process as claimed in any of the preceding claims wherein the pressure is from 140800 to 246400 kg/m² (200 psi to 350 psi).

5. A process as claimed in any of the preceding claims wherein the organic solvent is an aromatic, halogenated aromatic or a halogenated aliphatic hydrocarbon solvent.

6. A process as claimed in any of the preceding 20 claims wherein the organo aluminum halide is selected from the group consisting of alkyl aluminum halide or an alkyl aluminum sesquihalide, preferably with the halide function in the organo aluminum halide being chloride.

7. A process as claimed in any of the preceding claims wherein the phosphorous compound is selected from the group consisting of P[nBu]₃, PPh₃ and [Ph0]₂P[0][OH].

8. A process as claimed in any of the preceding claims wherein the oxygen compound is selected from the group consisting of tetrahydrofuran or 1,4 dioxane or a cyclic ether.

9. A process as claimed in any of the preceding claims wherein the sulphur compound is 2-mercaptobenzothiazole.

10. A process as claimed in any of the preceding claims wherein the said organoaluminum compound is reacted with the said titanium compound in a molar ratio of 1:8 to 1:18.

11. A process as claimed in any of the preceding claims wherein the said titanium compound is reacted with the said phosphorous, oxygen or sulphur compound in a molar ratio of 1:0.05 to 1:0.1.

## Patentansprüche

1. Verfahren zur Herstellung eines linearen α-Olefins durch Oligomerisieren von Ethylen in einem inerten organischen Lösungsmittel in Gegenwart eines Mischkatalysators umfassend Titanaryl/alkyloxid, Organoaluminiumhalogenid und gegebenenfalls einen Zusatz ausgewählt aus der Gruppe bestehend aus einer Phosphor-, Sauerstoff- oder Schwefelverbindung, bei einer Temperatur von 25 °C bis 150 °C und bei einem Druck, der ausreicht, um die Löslichkeit des zugeführten Ethylens aufrechtzuerhalten und um die Bildung von verzweigten Produkten zu verhindern, um das gewünschte lineare α-Olefin herzustellen.

2. Verfahren nach Anspruch 1, dadurch gekennzeichnet, daß der Katalysator die Formel besitzt
Ti[OR]ₙ[OR']₄₋ₙ-EtₓAl₂Cl₆₋ₓ - L
worin
x 2, 3 oder 4 ist,
Et Ethyl ist,
L abgeleitet ist von einer als Zusatz verwendeten Phosphor-, Sauerstoff- oder Schwefelverbindung, und
n = 4, und
R 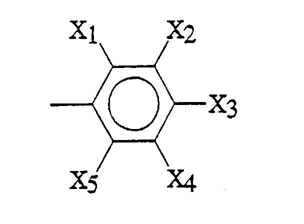
(worin
X₁ = CH₃; X₂=X₃=X₄=X₅ = H
X₂ = CH₃; X₁=X₃=X₄=X₅ = H
X₃ = CH₃; X₁=X₂=X₄=X₅ = H
X₄ = X₅=CH₃; X₂=X₃=X₄ = H)
oder
R 2-Ethylhexyl bedeutet, oder
n = 2, und R Triethanolamino oder Diethanolamino ist, und
R' Isopropyl ist.

3. Verfahren nach einem der vorhergehenden Ansprüche, dadurch gekennzeichnet, daß die Oligemerisierungsstemperatur 50 °C bis 150 °C beträgt.

4. Verfahren nach einem der vorhergehenden Ansprüche, dadurch gekennzeichnet, daß der Druck 140800 bis 246400 kg/m² (200 psi bis 350 psi) beträgt.

5. Verfahren nach einem der vorhergehenden Ansprüche, dadurch gekennzeichnet, daß das organische Lösungsmittel ein aromatischer, halogenierter aromatischer oder halogenierter aliphatischer Kohlenwasserstoff ist.

6. Verfahren nach einem der vorhergehenden Ansprüche, dadurch gekennzeichnet, daß das Organoaluminiumhalogenid ausgewählt ist aus der Gruppe bestehend aus einem Alkylaluminiumhalogenid oder einem Alkylaluminiumsesquihalogenid, wobei das Halogenid im organischen Aluminiumhalogenid vorzugsweise Chlorid ist.

7. Verfahren nach einem der vorhergehenden Ansprüche, dadurch gekennzeichnet, daß die Phosphorverbindung ausgewählt ist aus der Gruppe bestehend aus P[nBu]₃, PPh₃ und [PhO]₂P[O][OH].

8. Verfahren nach einem der vorhergehenden Ansprüche, dadurch gekennzeichnet, daß die Sauerstoffverbindung ausgewählt ist aus der Gruppe bestehend aus Tetrahydrofuran, 1,4-Dioxan oder einem cyclischen Ether.

9. Verfahren nach einem der vorhergehenden Ansprüche, dadurch gekennzeichnet, daß die Schwefelverbindung 2-Mercaptobenzothiazol ist.

10. Verfahren nach einem der vorhergehenden Ansprüche, dadurch gekennzeichnet, daß die Organoaluminiumverbindung mit der Titanverbindung in einem Molverhältnis von 1:8 bis 1:18 umgesetzt wird.

11. Verfahren nach einem der vorhergehenden Ansprüche, dadurch gekennzeichnet, daß die Titanverbindung mit der Phosphor-, Sauerstoff- oder Schwefelverbindung in einem Molverhältnis von 1:0,05 bis 1:0,1 umgesetzt wird.

## Revendications

1. Procédé de fabrication d'une alpha-oléfine linéaire qui comprend l'oligomérisation de l'éthylène dans un solvant, organique inerte en présence d'un catalyseur mixte comprenant un aryle de titane / oxyde d'alkyle, un halogénure d'organoaluminium et en option un additif sélectionné dans un groupe contenant un composé de phosphore, d'oxygène ou de soufre à une température de 25 °C à 150 °C et à une pression qui est suffisante pour maintenir la solubilité de l'éthylène introduit et pour éviter la formation de produits ramifiés pour produire l'alpha-oléfine linéaire souhaitée.

2. Procédé tel que revendiqué dans la revendication 1, caractérisé en ce que ledit catalyseur a la formule
Ti [OR]ₙ [OR']₄₋ₙ - EtₓAl₂Cl₆₋ₓ - L
dans laquelle
x vaut 2, 3 ou 4,
Et est le groupe éthyle,
L dérive d'un composé quelconque de phosphore, d'oxygène ou de soufre utilisé comme additif, et
n = 4, et
R est 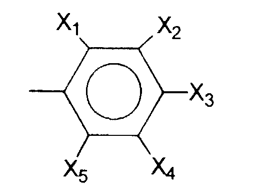
(dans laquelle
X₁ = CH₃ ; X₂ = X₃ = X₄ = X₅ = H
X₂ = CH₃ ; X₁ = X₃ = X₄ = X₅ = H
X₃ = CH₃ ; X₁ = X₂ = X₄ = X₅ = H
X₁ = X₅ = CH₃; X₂ = X₃ = X₄ = H)
ou
R est le groupe 2-éthylhexyle, ou
n = 2, et R est le groupe triéthanolamino ou diéthanolamino, et R' est le groupe isopropyle.

3. Procédé tel que revendiqué dans l'une quelconque des revendications précédentes, caractérisé en ce que la température d'oligomérisation est de 50 °C à 150 °C.

4. Procédé tel que revendiqué dans l'une quelconque des revendications précédentes, caractérisé en ce que la pression est de 140 800 à 246 400 kg/m² (200 psi à 350 psi).

5. Procédé tel que revendiqué dans l'une quelconque des revendications précédentes, caractérisé en ce que le solvant organique est un solvant aromatique, aromatique halogéné ou hydrocarbure aliphatique halogéné.

6. Procédé tel que revendiqué dans l'une quelconque des revendications précédentes, caractérisé en ce que l'halogénure d'organoaluminium est sélectionné dans le groupe constitué par un halogénure d'alkylaluminium ou un sesquihalogénure d'alkylaluminium, de préférence avec la fonction halogénure dans l'halogénure d'oganoaluminium étant le chlorure.

7. Procédé tel que revendiqué dans l'une quelconque des revendications précédentes, caractérisé en ce que le composé de phosphore est sélectionné dans le groupe constitué par P[nBu]₃, PPh₃ et [PhO]₂P[O][OH].

8. Procédé tel que revendiqué dans l'une quelconquedes revendications précédentes, caractérisé en ce que le composé d'oxygène est sélectionné dans le groupe constitué par le tétrahydrofurane ou le 1,4-dioxane ou un éther cyclique.

9. Procédé tel que revendiqué dans l'une quelconque des revendications précédentes, caractérisé en ce que le composé de soufre est le 2-mercaptobenzotriazole.

10. Procédé tel que revendiqué dans l'une quelconque des revendications précédentes, caractérisé en ce que ledit composé organoaluminium est mis à réagir avec ledit composé de titane selon un rapport molaire de 1 : 8 à 1 : 18.

11. Procédé tel que revendiqué dans l'une quelconque des revendications précédentes, caractérisé en ce que ledit composé de titane est mis à réagir avec ledit composé de phosphore, d'oxygène ou de soufre selon un rapport molaire de 1 : 0,05 à 1 : 0,1.
